# EUROPEAN PATENT APPLICATION

(11) **EP 4 635 561 A1**
(43) Date of publication of application: **22.10.2025**
(21) Application number: 24170250.5
(22) Date of filing: 15.04.2024
(51) Int. Cl.: A61N 5/06

(54) **APPARATUS FOR INDUCING PHOTOBIOMODULATION IN A USER**

(71) Applicant: Sunled Life Science B.V., 1098XG Amsterdam (NL)
(72) Inventor: BERENDS, Anne Claire, 1098 XG Amsterdam (NL); KRAMES, Michael, 1098 XG Amsterdam (NL); HILGERINK, Tom, 1098 XG Amsterdam (NL)
(74) Representative: Hoyng Rokh Monegier B.V.

(57) **Abstract**

Apparatus for providing radiation for stimulating photobiomodulation (PBM) in a user comprising a plurality of emitters for emitting radiation in the near-infrared spectrum, a receiver for receiving radiation in the near-infrared spectrum, and an emitter driver circuit configured to generate driving currents for energizing the emitters. The apparatus is configured to energize a first portion of the emitters to generate radiation in a first part of the near-infrared spectrum for detecting the user, with a driving current according to a first activation sequence, and to activate the receiver for receiving at least a reflected portion of the radiation from at least the first portion of the emitters for detecting the user. The apparatus is also configured to energize a second portion of the emitters to generate radiation in a second part of the near-infrared spectrum for stimulating PBM in the user, with a driving current according to a second activation sequence. In the apparatus, the first portion of the emitters is different from the second portion of the emitters, and/or the first part of the near-infrared spectrum is different from the second part of the near-infrared spectrum, and/or the first activation sequence of the driving current is different from the second activation sequence of the driving current.

## Description

### TECHNICAL FIELD

The invention relates generally to a method and system for providing radiation to induce a photobiomodulation (PBM) response in a user.

### BACKGROUND ART

Photobiomodulation (PBM) involves irradiating a living organism at certain energy/power levels to induce biological or biochemical responses. The irradiation may be in the visible spectrum, such as red light, or in the non-visible spectrum, such as near-infrared (NIR). There has been a significant amount of research about the medical benefits of employing PBM therapy to provide physical and psychological benefits.

PBM may be induced in a user by exposing the user to suitable radiation over a sufficiently long time period. This may be accomplished by including a suitable radiation source in electrical equipment such as overhead lighting fixtures, where it may be expected that the user is exposed to the radiation for several hours in a day. In this situation, it is beneficial to pulse the radiation source to avoid overdosing the user and reduce component count and driver power, while still providing sufficient irradiance to the user's skin to induce a PBM response in the user.

PBM may also be induced in a user by exposing the user to suitable radiation over a shorter time period, such as for an hour or less. In this situation, overdosing is generally not an issue and the radiation source may be energized continuously (i.e. not pulsed) or pulsed at a relatively high duty cycle to provide sufficient dose to the user in a shorter time. However, adjusting the radiation to a suitable intensity and directing it effectively to irradiate the user's skin can be difficult when the user is moving about during the exposure period. This makes it difficult to set the correct radiation intensity, to utilize a narrow radiation beam, and estimate the dose received by the user and adjust the radiation accordingly.

Therefore, there is a need to overcome the abovementioned disadvantages of the currently available apparatuses and methods.

### SUMMARY OF THE INVENTION

It is desirable to provide an apparatus that is easy to use, energy efficient, cost effective and yet emits an amount of radiation sufficient to induce a PBM response. Accordingly, the invention provides an apparatus and method for enhancing the health and safety of users, in particular vehicle occupants, by inducing PBM.

The invention provides an apparatus and method for providing radiation for stimulating PBM in a user. The apparatus includes a radiation source comprising a plurality of emitters for emitting radiation in the near-infrared spectrum, a receiver for receiving radiation in the near-infrared spectrum, and an emitter driver circuit configured to generate driving currents for energizing the emitters. The apparatus is configured to energize a first portion of the emitters to generate radiation in a first part of the near-infrared spectrum for detecting the user, with a driving current according to a first activation sequence, and to activate the receiver for receiving at least a reflected portion of the radiation from at least the first portion of the emitters for detecting the user. The apparatus is also configured to energize a second portion of the emitters to generate radiation in a second part of the near-infrared spectrum for stimulating PBM in the user, with a driving current according to a second activation sequence. In the apparatus, the first portion of the emitters is different from the second portion of the emitters, and/or the first part of the near-infrared spectrum is different from the second part of the near-infrared spectrum, and/or the first activation sequence of the driving current is different from the second activation sequence of the driving current. In some embodiments, the first portion and/or the second portion of the emitters comprises a single emitter.

In one embodiment, the first portion of the emitters is the same as the second portion of the emitters. In this way, the same emitters may be used for both user detection and for inducing PBM in the user, the emitters emitting radiation in the near-infrared spectrum at wavelengths which are suitable for both purposes. The emitters may be light-emitting diodes (LEDs) or laser diodes (e.g., vertical-cavity surface-emitting lasers, or VCSELs), for example. In this embodiment, the PBM function may be added to a device with user detection, such as a driver monitoring system, by using the emitters already present in the device and without requiring additional emitters.

In another embodiment, a peak wavelength of the radiation emitted by the first portion of emitters is different from a peak wavelength of the radiation emitted by the second portion of emitters. In one implementation of this embodiment, the peak wavelength of the radiation emitted by the first portion of emitters may differ by at least 50 nm from a peak wavelength of the radiation emitted by the second portion of emitters. In another implementation, the first part of the near-infrared spectrum does not overlap with the second part of the near-infrared spectrum. In one implementation, the first portion of emitters are adapted to emit radiation having a peak wavelength above 900 nm, and the second portion of emitters are adapted to emit radiation having a peak wavelength below 900 nm. In these ways, different wavelengths are used for the user detection function and for the PBM inducing function, so that the radiation used for each function does not interfere with the other function. In one implementation, the receiver is modified with a filter (e.g. a band pass filter or a long pass filter or a short pass filter) to make it more wavelength specific and reduce interference.

In one embodiment, the first activation sequence of the driving current differs from the second activation sequence of the driving current so that the energizing the first portion of emitters does not overlap in time with energizing the second portion of emitters. In this way, the first portion of emitters are not energized and emitting radiation at the same time as the second portion of emitters, so that the radiation for user detection is emitted at different times from the radiation for inducing PBM. By energizing the two portions of emitters at different times, any interference to the user detection function caused by the PBM inducing radiation can be reduced. In one implementation, the receiver is blanked (e.g. deactivated) during the periods when the PBM inducing radiation is emitted.

In one embodiment, the receiver is not activated during the second activation sequence of the driving current. In this way, the receiver is not activated when the second portion of emitters is energized and emitting the radiation for inducing PBM, so that any interference to the user detection function caused by the PBM inducing radiation can be reduced or avoided.

These embodiments thus provide various means for including a PBM inducing function in a user detection device, such as a driver monitoring system, without interfering with the user detection function.

In one embodiment, the apparatus is configured to adjust the energizing of the second portion of the emitters based at least in part on the radiation emitted by the first portion of the emitters. The radiation emitted by the first portion of emitters which is used for driver detection may provide some PBM inducing effect in the user. The adjustment of energizing of the second portion of the emitters may be made based on the wavelength, intensity and/or duration of the radiation emitted from the first portion of emitters.

In one embodiment, the apparatus is configured to generate a signal in dependence on a distance to the user based on the reflected radiation received by the receiver and adjust the energizing of the second portion of the emitters based at least in part on the signal. The radiation received by the receiver may be used by the apparatus to detect how far the user is from the apparatus, e.g. the distance between the user's face or other part of the user and the receiver or some other location used by the apparatus as a reference point. The apparatus may be configured to detect the presence of the user within a working range of the radiation emitted by the second portion of the emitters, i.e. detecting when the user is at a distance suitable for inducing PBM in the user by the apparatus. The apparatus may be configured to deactivate the second portion of emitters when the user is too close to the emitter, e.g. to prevent retinal damage and/or to adhere to eye safety standards. The apparatus may be configured to deactivate the second portion of emitters when the user is too far away from the emitter, e.g. because the irradiance is too low to induce a PBM effect in the user.

In one embodiment, the apparatus is configured to recognize a face of the user, and adjust the energizing of the second portion of the emitters based at least in part on the recognition of the face. The apparatus may be configured to recognize the face of a user, e.g. using facial recognition, and activate the second portion of emitters accordingly. This may include recognizing the face of a particular user, rather than generally recognizing any face. In this way, the apparatus may be configured to personalize the PBM dosage to a particular user, e.g. by taking into account the characteristics of the user and/or previous PBM dose provided by the apparatus or otherwise. The apparatus may comprise a communication unit for receiving data from mobile communication devices of the user. This may be used to recognize a particular user and personalize PBM dosage for that user.

In one embodiment, the apparatus comprises a detection unit configured to detect a position of the user or a portion of the user based on the reflected radiation received by the receiver and is configured to adjust a direction at which the radiation from the second portion of the emitters is emitted from the apparatus based on the detection. For example, the radiation may be directed towards the face of the user, or the face and neck area of the user. The detection unit may be configured to adjust a radiation pattern of the radiation from the second portion of the emitters which is emitted from the apparatus based on the detection. For example, the apparatus may be configured to form the radiation emitted by the second portion of the emitters into a non-circular beam shape, and/or may be configured to adjust the direction and/or spread angle of the radiation beam emitted by the second portion of the emitters. The apparatus may comprise an optical element comprising one or more lenses, and/or one or more mirrors, and/or one or more diffraction optical elements, wherein the apparatus is configured to move and/or rotate a part of the optical element to adjust the direction and/or radiation pattern of the radiation beam. The apparatus may be configured with a least some of the second portion of emitters being driven by separate driving currents, and may be configured to adjust the driving currents for second portion of emitters in reaction to an input from the detection unit, to adjust a direction and/or radiation pattern of the radiation beam. The apparatus may be configured to adjust the radiation emitted by the second portion of the emitters to have a beam angle of between 4-60° FAHP. By adopting one or more of these features, the apparatus may be configured to generate a (narrow) radiation beam to induce PBM in the user, by focusing energy of the radiation on the user in a known position. The direction and radiation pattern of the radiation may be adjusted to illuminate the face of the user, or the face and neck area of the user, e.g. to illuminate at least 250 cm2 of the skin of the user.

In one embodiment, the apparatus is configured so that the second activation sequence of the driving current energizes the second portion of emitters such that a predetermined threshold irradiance is surpassed for a period of at least 0.1 ms during each period when the second portion of the emitters is energized, wherein the predetermined threshold irradiance is 1 mW/cm2 or more. In this way, radiation is generated by the second portion of emitters sufficient to induce PBM in the user.

In one embodiment, the apparatus is configured to adjust the energizing of the second portion of the emitters based at least in part on an estimated dose of radiation delivered by the apparatus to the user. In this way, the apparatus can keep track of the dose supplied to the user and reduce or stop generating the PBM radiation once a predetermined dose has been delivered to the user, e.g. where the predetermined dose is between 1 and 50 J/cm2.

In one embodiment, the apparatus is mounted in a vehicle for stimulating PBM in an occupant of a vehicle. For example, the apparatus may be mounted in or on the dashboard of the vehicle or may be integrated in a driver monitoring system or a cabin display system for the driver or passenger in the vehicle.

In one embodiment, red glow of the emitters is prevented. This can be done by using emitters that do not emit radiation in the visible portion of the spectrum. For example, this may be achieved using long wavelength LEDs without a tail in the visible part of the spectrum (e.g. 980 nm LEDs), or by using VCSELs, or by covering the emitters with a material that is not transparent for visible light (e.g. a long pass filter or a band pass filter).

In another aspect, the invention provides a method for providing radiation for stimulating PBM in a user, the method comprising providing a radiation source comprising a plurality of emitters for emitting radiation in the near-infrared spectrum, providing a receiver for receiving radiation in the near-infrared spectrum, and providing an emitter driver circuit configured to generate driving currents for energizing the emitters. The method is performed by energizing a first portion of the emitters to generate radiation in a first part of the near-infrared spectrum for detecting the user, with a driving current according to a first activation sequence, activating the receiver for receiving at least a reflected portion of the radiation from at least the first portion of the emitters for detecting the user, and energizing a second portion of the emitters to generate radiation in a second part of the near-infrared spectrum for stimulating PBM in the user, with a driving current according to a second activation sequence. The first portion of the emitters is different from the second portion of the emitters, and/or the first part of the near-infrared spectrum is different from the second part of the near-infrared spectrum, and/or the first activation sequence of the driving current is different from the second activation sequence of the driving current.

In another aspect, the invention provides a method to provide PBM to an occupant of a vehicle.

### BRIEF DESCRIPTION OF THE DRAWINGS

Embodiments will now be described, by way of example only, with reference to the accompanying schematic drawings in which corresponding reference symbols indicate corresponding parts, and in which:
FIG. 1 is an illustration of an apparatus fitted in a vehicle for providing radiation for stimulating photobiomodulation in a user;
FIG. 2 is a schematic block diagram of an embodiment of an apparatus providing radiation for stimulating photobiomodulation in a user; and
FIG. 3 is a timing diagram of driving currents for energizing emitters for an apparatus for providing radiation for stimulating photobiomodulation in a user.

The figures are meant for illustrative purposes only, and do not serve as restriction of the scope or the protection as laid down by the claims.

### DESCRIPTION OF ILLUSTRATIVE EMBODIMENTS

The following is a description of certain embodiments of the invention, given by way of example only and with reference to the drawings.

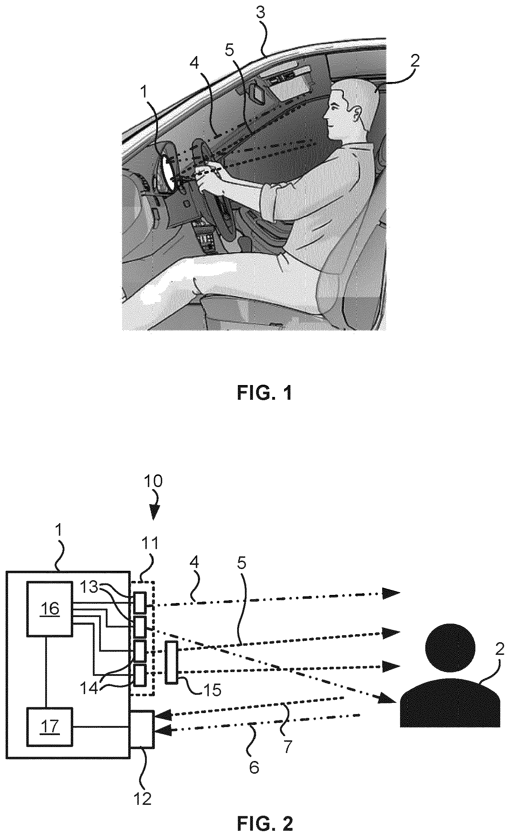

FIG. 1 is an illustration of an apparatus 1 for providing radiation for stimulating photobiomodulation (PBM) in a user 2, where the apparatus is fitted in a vehicle 3. The apparatus 1 includes a radiation source comprising a plurality of emitters for emitting radiation 4 in the near-infrared spectrum for detecting the user 2 and emitting radiation 5 in the near-infrared spectrum for inducing PBM in the user 2.

The apparatus is mounted in a vehicle 3 and may be used for detecting the presence and position of the driver or other occupant of the vehicle, and for stimulating PBM in the driver or other occupant of the vehicle. The apparatus may be mounted in or on the dashboard of the vehicle as shown in the example in FIG. 1, for example in a driver monitoring system. The apparatus may also be integrated in a display system for the driver or passenger in the vehicle, such as an entertainment or infotainment system. The apparatus may be employed in any type of vehicle, such as an automobile, train, airplane, ship etc.

FIG. 2 is a schematic block diagram of the apparatus 1, which includes a radiation source 10 comprising the emitters 11 for emitting radiation in the near-infrared spectrum, a receiver 12 for receiving radiation in the near-infrared spectrum, and an emitter driver circuit 16 configured to generate driving currents for energizing the emitters.

A first portion of the emitters 13 generates radiation 4 in a first part of the near-infrared spectrum for detecting the user 2, e.g. for detecting the position of the user with respect to the apparatus. The receiver 12 is configured for receiving a reflected portion of the radiation 4 for detecting the user 2. A detection unit 17 included in the apparatus for receiving a detection signal from receiver 12 for detecting the user 2, e.g. detecting the presence of the user, the distance of the user from the apparatus, the location of the user within the vehicle, the sitting position of the user, or other similar information regarding the user. The receiver 12 and detection unit 17 may be configured to recognize the face of the user, e.g. using facial recognition technology. The detection unit may be configured to detect a face, or to detect a particular person's face (to differentiate between different users), and may be configured to detect signs of drowsiness or inattention, e.g. of a driver or pilot.

A second portion of the emitters 14 generates radiation 5 in a second part of the near-infrared spectrum for stimulating PBM in the user 2. The apparatus may include an optical element 15 comprising one or more lenses, mirrors, and/or diffraction optical elements, and the apparatus configured to move or rotate a part of the optical element 15 to adjust the direction and/or radiation pattern of the radiation beam 5, based on a signal form the detection unit 17. Also, the individual emitters that generate radiation 5 may be driven by separate driving currents, so that the driving currents may be adjusted by the apparatus to adjust the direction and/or radiation pattern of the radiation beam 5. The radiation 5 is preferably a narrow beam, e.g. with a beam angle of between 4-60° FAHP, which is directed towards the face and neck area of the user 2, preferably illuminating at least 250 cm² of the skin of the user 2.

The first portion of emitters 13 is energized by a driving current according to a first activation sequence to emit pulsed radiation 4 for detection of the user 2. The second portion of emitters 14 is energized by a driving current according to a second activation sequence to emit radiation 5 for stimulating PBM in the user 2. The driving current for the first and second portions of emitters are generated by the emitter driver circuit 16.

In one embodiment, the same emitters 11 are used to generate the radiation 4 for detecting the user and the radiation 5 for inducing PBM in the user, i.e. the first portion of the emitters is the same as the second portion of the emitters. This arrangement may be preferred when the same wavelength radiation is used for both detecting the user and inducing PBM in the user. In this arrangement, the radiation dose for inducing PBM in the user may be partly achieved by the radiation 4 that is also used for detecting the user 2, and partly by the radiation 5. The receiver 12 may be activated only during periods when radiation 4 is generated, or only during periods when radiation 5 is not generated, to avoid any disturbance to the user detection function as a result of the additional radiation 5 generated for the PBM-inducing function.

In another embodiment, the emitters used to generate radiation 4 are different from the emitters used to generate radiation 5, i.e. the first portion of the emitters 13 is different from the second portion of the emitters 14. This arrangement may be necessary when different wavelength radiation is used for detecting the user than for inducing PBM in the user, but it can also be used where the two sets of emitters both generate radiation having the same wavelength (e.g. radiation 4 and radiation 5 have the same wavelength).

The emitters 11 may be LEDs or VCSELs, for example. Infrared VCSELs or high-power LEDs are used in driver monitoring systems (DMS). For example, user detection may involve facial recognition of the user using short duration, high current pulses with high near-infrared light energy content to create accurate spatial maps of the user even in difficult conditions, e.g. in strong sunlight and when the user is wearing sunglasses. DMS systems that use pulsed LEDs or VCSELs may enable the addition of PBM inducing functionality to the DMS by using the already existing hardware with only small adaptations required.

In one embodiment, the same wavelength of radiation may be used for detecting the user (radiation 4) and for inducing PBM in the user (radiation 5). In another embodiment, different wavelengths may be used for these two purposes. For example, the peak wavelength of radiation 4 (e.g. the peak being the wavelength having the highest intensity or power) may be different from the peak wavelength of radiation 5. For example, the peak wavelength for the radiation 4 may differ by at least 50 nm from that of the radiation 5, and/or there may be no overlap of the range of wavelengths of radiation 4 and the range of wavelengths of radiation 5.

In one implementation, radiation 4 has a peak wavelength above 900 nm, and radiation 5 has a peak wavelength below 900 nm. For example, the use of radiation 4 for detecting the user 2 centered around 940 nm (e.g. with a peak intensity or power at 940 nm) may offer less interference from ambient sunlight and reduce the visible red glow which is apparent at lower frequencies. However, the quantum efficiency of the image sensors in the receiver 12 may drop when a higher wavelength such as 940 nm is used for detecting the user. The use of radiation 5 for stimulating PBM in user 2 may be centered around 850 nm (e.g. with a peak intensity or power at 850 nm). The use of different wavelength radiation for the user detection function as opposed to the PBM inducing function has the advantage that the radiation used for one function does not interfere with the other function. The achieved reduction of avoidance of interference can be further enhanced by applying filters to the receiver.

In one embodiment, the radiation 4 and radiation 5 are generated at different times, to avoid the PBM-inducing function interfering with the user detection function of the apparatus. This can be achieved by energizing the emitters 11 to generate radiation 4 for detecting the user at different times from energizing the emitters 11 to generate radiation 5. The receiver 12 can then be activated to receive a reflected portion of radiation 4 during the periods when radiation 4 is generated, and deactivated during the periods when radiation 5 is generated. This avoids the situation in which radiation 5 (used for inducing PBM in the user) interferes with the user detection function associated with radiation 4.

In one embodiment, a first driving current is used to energize emitters 13 to generate radiation 4 wherein the first driving current is according to a first activation sequence, and a second driving current having a different second activation sequence is used for energizing the emitters 14 to generate radiation 5. In addition or instead, the receiver 12 may be activated only during periods when radiation 4 is being generated or when radiation 5 is not being generated.

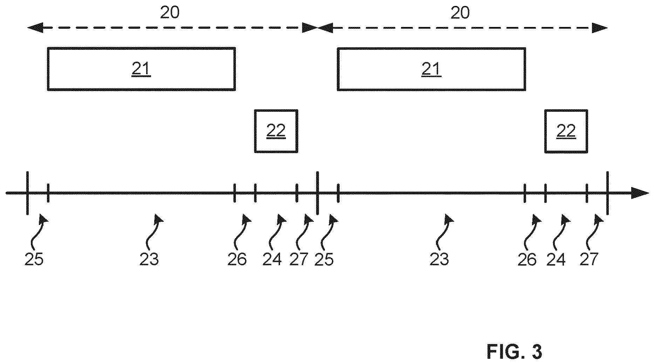

FIG. 3 illustrates a timing diagram showing an example in which (at least a portion of) the emitters 11 are energized by driving current according to a first activation sequence 21 during periods 23 for generating radiation 4, and are energized by driving current according to a second activation sequence 22 during periods 24 for generating radiation 5. The activation sequences may repeat periodically in each time frame 20. Each frame may include for example an emitter turn-on period 25, a read-out period 26 during which an output is generated by the receiver 12, and a reset period 27. The period 24 may be a blanking period for the user detection function of the apparatus (e.g. during which the receiver 12 is deactivated), and during this period the second activation sequence 22 energizes the emitters 11 to generate radiation 5 for the PBM inducing function.

The first activation sequence 21 typically comprises a series of short pulses during period 23 (e.g. using a pulsed driving current), and the second activation sequence 22 typically comprises a series of short pulses during period 24 (e.g. using a pulsed driving current). Alternatively, the second activation sequence 22 may comprise continuous energizing of the emitters 14 during period 24. By energizing the two portions of emitters at different times, any interference to the user detection function caused by the PBM inducing radiation can be reduced or avoided.

In one embodiment, the receiver is not activated during the second activation sequence of the driving current. In this way, the receiver is not activated when the second portion of emitters is energized and emitting the radiation for inducing PBM, so that any interference to the user detection function caused by the PBM inducing radiation can be reduced.

These embodiments thus provide various means for including a PBM inducing function in a user detection device, such as a driver monitoring system, without interfering with the user detection function.

The apparatus may be configured to take into account the PBM-inducing effect of the radiation 4 and adjust the timing or intensity of the second driving current for generating radiation 5. The apparatus may also use information regarding the proximity of the user to the apparatus and position of the user to adjust the timing or intensity of the second driving current for generating radiation 5, and/or to direct the radiation 5 towards the user, and/or shut off the radiation 5.

The foregoing description is intended to be illustrative, not limiting. It will be apparent to the person skilled in the art that alternative and equivalent embodiments of the invention can be conceived and reduced to practice, without departing from the scope of the invention described in the claims set out below.

## Claims

1. An apparatus for providing radiation for stimulating photobiomodulation in a user, the apparatus comprising:
a radiation source comprising a plurality of emitters for emitting radiation in the near-infrared spectrum;
a receiver for receiving radiation in the near-infrared spectrum; and
an emitter driver circuit configured to generate driving currents for energizing the emitters;
wherein the apparatus is configured to:
energize a first portion of the emitters to generate radiation in a first part of the near-infrared spectrum for detecting the user, with a driving current according to a first activation sequence;
activate the receiver for receiving at least a reflected portion of radiation from at least the first portion of the emitters for detecting the user; and
energize a second portion of the emitters to generate radiation in a second part of the near-infrared spectrum for stimulating photobiomodulation in the user, with a driving current according to a second activation sequence;
wherein:
(a) the first portion of the emitters is different from the second portion of the emitters, and/or
(b) the first part of the near-infrared spectrum is different from the second part of the near-infrared spectrum, and/or
(c) the first activation sequence of the driving current is different from the second activation sequence of the driving current.

2. The apparatus of claim 1, wherein the first portion of the emitters is the same as the second portion of the emitters.

3. The apparatus of any one of the preceding claims, wherein a peak wavelength of the radiation emitted by the first portion of emitters is different from a peak wavelength of the radiation emitted by the second portion of emitters.

4. The apparatus of claim 3, wherein a peak wavelength of the radiation emitted by the first portion of emitters differs by at least 50 nm from a peak wavelength of the radiation emitted by the second portion of emitters.

5. The apparatus of claim 3, wherein the first portion of emitters are adapted to emit radiation having a peak wavelength above 900 nm, and the second portion of emitters are adapted to emit radiation having a peak wavelength below 900 nm.

6. The apparatus of any one of the preceding claims, wherein the first activation sequence of the driving current differs from the second activation sequence of the driving current so that energizing the first portion of emitters does not overlap in time with energizing the second portion of emitters.

7. The apparatus of any one of the preceding claims, wherein the receiver is not activated during the second activation sequence of the driving current.

8. The apparatus of any one of the preceding claims, wherein the apparatus is configured to adjust the energizing of the second portion of the emitters based at least in part on the radiation emitted by the first portion of the emitters.

9. The apparatus of any one of the preceding claims, wherein apparatus is configured to generate a signal in dependence on a distance between the apparatus and the user based on the reflected radiation received by the receiver, and adjust the energizing of the second portion of the emitters based at least in part on the signal.

10. The apparatus of any one of the preceding claims, wherein the apparatus is configured to recognize a face of the user, and adjust the energizing of the second portion of the emitters based at least in part on the recognition of the face.

11. The apparatus of any one of the preceding claims, wherein the apparatus is configured to detect a position of a portion of the user based on the reflected radiation received by the receiver, and is configured to adjust a direction at which the radiation from the second portion of the emitters is emitted from the apparatus.

12. The apparatus of any one of the preceding claims, wherein the apparatus is configured to detect a position of a portion of the user based on the reflected radiation received by the receiver, and is configured to adjust a radiation pattern of the radiation from the second portion of the emitters which is emitted from the apparatus.

13. The apparatus of any one of the preceding claims, wherein the second activation sequence of the driving current energizes the second portion of emitters such that a predetermined threshold irradiance is surpassed for a period of at least 0.1 ms during each period of when the second portion of emitters in energized, wherein the predetermined threshold irradiance is 1 mW/cm² or more.

14. The apparatus of any one of the preceding claims, wherein the apparatus is configured to adjust the energizing of the second portion of the emitters based at least in part on an estimated dose of radiation delivered by the apparatus to the user.

15. The apparatus of any one of the preceding claims, wherein the apparatus is mounted in a vehicle for providing radiation for stimulating photobiomodulation in an occupant of a vehicle.

16. A method for providing radiation for stimulating photobiomodulation in a user, the method comprising:
providing a radiation source comprising a plurality of emitters for emitting radiation in the near-infrared spectrum;
providing a receiver for receiving radiation in the near-infrared spectrum; and
providing an emitter driver circuit configured to generate driving currents for energizing the emitters;
energizing a first portion of the emitters to generate radiation in a first part of the near-infrared spectrum for detecting the user, with a driving current according to a first activation sequence;
activating the receiver for receiving at least a reflected portion of the radiation from at least the first portion of the emitters for detecting the user; and
energizing a second portion of the emitters to generate radiation in a second part of the near-infrared spectrum for stimulating photobiomodulation in the user, with a driving current according to a second activation sequence;
wherein:
(a) the first portion of the emitters is different from the second portion of the emitters, and/or
(b) the first part of the near-infrared spectrum is different from the second part of the near-infrared spectrum, and/or
(c) the first activation sequence of the driving current is different from the second activation sequence of the driving current.
